# EUROPÄISCHE PATENTSCHRIFT

(11) **EP 1 390 492 B1**
(45) Veröffentlichungstag und Bekanntmachung des Hinweises auf die Patenterteilung: **16.07.2008**
(21) Anmeldenummer: 02745336.4
(22) Anmeldetag: 31.05.2002
(51) Int. Cl.: C12N 15/10, B01L 3/00

(54) **GERICHTETE EVOLUTION VON BIOMOLEKÜLEN UNTER DER VERWENDUNG VON MIKROSTRUKTUREN**
DIRECTED EVOLUTION OF BIOMOLECULES BY USING MICROSTRUCTURES
L'EVOLUTION ORIENTEE DE BIOMOLECULES AVEC UTILIZATION DE MICROSTRUCTURES

(30) Priorität: 31.05.2001 EP 01113331
(43) Veröffentlichungstag der Anmeldung: 25.02.2004
(73) Patentinhaber: Direvo Biotech AG, 50829 Köln (DE)
(72) Erfinder: RARBACH, Markus, 50829 Köln (DE); KETTLING, Ulrich, 50829 Köln (DE); STEPHAN, Jens, 50829 Köln (DE); KOLTERMANN, Andre, 50829 Köln (DE)
(74) Vertreter: Helbing, Jörg
(86) Internationale Anmeldenummer: PCT/EP2002/005971
(87) Internationale Veröffentlichungsnummer: WO 2002/097130

(56) Entgegenhaltungen:
- WO-A-00/56872
- DE-A- 19 950 385
- US-A- 6 130 098
- US-B1- 6 207 031

### SEQUENZPROTOKOLL

<110> DIREVO Biotech AG
<120> Mikrostrukturen und deren Verwendung für die gerichtete Evolution von Biomelekülen
<130> 021342wo/JH/ml
<140>
   <141>
<160> 2
<170> PatentIn Ver. 2.1
<210> 1
   <211> 66
   <212> DNA
   <213> Künstliche Sequenz
<220>
   <223> Beschreibung der künstlichen Sequenz: Primer
<400> 1
   atggctaatg accgagaata gggatccgaa ttcaatattg gtacctacgg gctttgcgct 60
   cgtatc                                                            66
<210> 2
   <211> 66
   <212> DNA
   <213> Künstliche Sequenz
<220>
   <223> Beschreibung der künstlichen Sequenz: Primer
<400> 2
   gatacgagcg caaagcccgt aggtaccaat attgaattcg gatccctatt ctcggtcatt 60
   agccat                                                            66

## Beschreibung

Die vorliegende Erfindung betrifft ein Verfahren für die gerichtete Evolution von Biomolekülen.

### Hintergrund der Erfindung

In Verfahren der gerichteten molekularen Evolution werden aus Bibliotheken, die eine Vielzahl von Varianten eines Biomoleküls enthalten, Varianten selektiert, die einem festgelegten Entwicklungsziel entsprechen. Wiederholung von Variation, Amplifikation und Selektion von Varianten werden optimierte Biomoleküle generiert.

Verfahren der gerichteten molekularen Evolution beruhen primär auf der Erzeugung einer großen Zahl von DNA-Varianten (Genotyp-Bibliothek). Ausgehend von einer solchen Bibliothek von Genotypen werden die korrespondierenden Genprodukte hergestellt, bzgl. Ihrer Eigenschaften (Phänotyp) durchmustert und entsprechend selektiert. Zur Untersuchung erweisen sich Screeningverfahren aufgrund ihrer Flexibilität und generellen Einsetzbarkeit gegenüber anderen, beispielsweise wachstumsgekoppelten Selektionsverfahren als besonders vorteilhaft.

Screeningverfahren basieren auf der räumlichen Isolierung der Genotypvarianten. Diese Isolierung der Genotypen sichert sowohl die getrennte Messbarkeit von Eigenschaften der unterschiedlichen Phänotypen als auch die Zuordnung von Genotyp und Phänotyp, die zur Selektion und Amplifikation optimaler Genotypen unerlässlich ist. Da die Zahl der zu untersuchenden Varianten sehr groß sein kann, wird die Segregierung der Genotypen in bisher durchgeführten Verfahren in der Regel in Probenträgern realisiert, die eine Vielzahl von Probenkompartimenten zusammenfassen. Handelsübliche Probenträger tragen beispielweise 96, 384 oder 1536 Probenkompartimente. Die Zahl der Probenkompartimente wird dabei möglichst groß gewählt, um die Zahl der benötigten Probenträger und die Menge der benötigten Assayreagenzien zu begrenzen. Limitierend in der Entwicklung von Probenträgern mit einer noch größeren Zahl von Probenkompartimenten ist die Handhabung der geringen Flüssigkeitsmengen in diesen Kompartimenten. Viskositäts- und Oberflächenspannungseinflüsse sowie Verdunstung von fluiden Proben, die in größeren Volumina von untergeordneter Bedeutung sind, beschränken diese Vorgehensweise stark.

Aufgabe der Erfindung ist es, ein Screeningverfahren für die gerichtete molekulare Evolution bereitzustellen, das die genannten Nachteile Probenträgerorientierter Screeningverfahren vermeidet.

Prinzipiell ist ein Verfahren mit vergleichbarer Aufgabe aus WO9535492 bekannt. Das beschriebene Verfahren ist geeignet, Probenbestandteile eines fluiden, in einer Kapillare transportierten Probengemisches entsprechend ihrer Eigenschaften zu trennen. Nachteilig an diesem Verfahren ist, dass keine Möglichkeit zur Reduzierung des diffusiven Stofftransports innerhalb des Fluidstroms vorgesehen ist. Dieser Mangel verhindert die Nutzung sehr kleiner Probenkompartimente, da insbesondere bei kleinen Abmessungen der diffusive Verlust von Probenbestandteilen die Durchführbarkeit der angestrebten Reaktionen im Probenkompartiment verhindert. Weiter erfordert die technische Realisierung des Verfahrens hohe Ansprüche an die mechanische Positionierung der Komponenten. Solche Lösungen erweisen sich häufig als instabil und fehleranfällig.

Des Weiteren ist aus der DE-A-19950385 ein *in-vivo*-Screeningverfahren bekannt, welches die Identifizierung von an sich nichtselektionierbaren Aktivitäten in einer Zielzelle ermöglicht. Die zu untersuchende Nukleinsäuresequenz wird zusammen mit einem Reportervektor durch Transfektion in diese Zielzellen eingeschleust. Die aus dem Reporter resultierende Aktivität in den Zielzellen oder in deren Kulturüberstand wird als Maß für die nichtselektionierbare Aktivität der untersuchten Nukleinsäuresequenz und deren Identifizierung herangezogen.Es ist jedoch bekannt, dass der Einsatz von Verfahren zur *in-vivo* Expression durch einige Faktoren limitiert ist. So können intrazelluläre Nukleasen bzw. Proteasen den eingebrachten Genotyp bzw. das Genprodukt zerstören. Die exprimierten Genprodukte können toxisch oder inhibierend auf die Wirtszellen wirken und damit deren Effektivität beeinträchtigen. Die Genprodukte können zudem in unlöslicher Form bzw. biologisch unwirksamer Form sei als "inclusion body" exprimiert werden.

Dagegen ist die zellfreie in-vitro-Expression an keine zellulären Kontrollmechanismen gebunden, ermöglicht ohne Isolierungsschritte einen direkten Zugang zu den exprimierten Genprodukten. Zudem ist die Darstellung von artifiziellen Genprodukten durch die Einbindung von modifizierten, nicht proteinogenen Aminosäuren möglich.

Die vorliegende Erfindung beschreibt ein. Screeningverfahren, das unter, Verwendung von geeigneten Mikrostrukturen zur selektiven Identifizierung von Genotypen basierend auf zellfreier *in-vitro* Expression geeignet ist.

### Kurzbeschreibung der Erfindung

Es wurde gefunden, dass das Screening eines Proben-Ensembles sowie die Selektion oder Sortierung einzelner Proben aus diesem Ensemble in einem *in-vitro*-Verfahren durch Mikrostrukturierungstechniken gefertigten Kanalstrukturen erfolgen kann. Die Aufteilung in einzelne Proben erfolgt in diesen Mikrostrukturen durch Segregierung mittels verschiedener fluider Phasen. Die vorliegende Erfindung betrifft somit
(1) ein Verfahren zur zellfreien (d.h. *in-vitro*) Selektion von Genotyp-Varianten aus Genotyp-Bibliotheken in einer Mikrostruktur, umfassend die folgende Abfolge von Reaktionsschritten:
   (a) Zusammenführen eines Testfluids, umfassend eine Genotypen-Bibliothek in einer exprimierbaren Form und für die zellfreie Expression geeignete Expressionshilfsstoffe, und eines Trennfluids in der Mikrostruktur, sodass einzelne Kompartimente des Testfluids entstehen;
   (b) Transportieren der Kompartimente durch die Mikrostruktur, wobei die Expression des Genotyps in den Phänotyp in den Kompartimenten erfolgt,
   (c) Detektieren des Phänotyps in den Kompartimenten und
   (d) Selektieren der Kompartimente entsprechend ihrer Phänotypen.

### Kurzbeschreibung der Figuren

Wesentliche Funktionen und Eigenschaften des erfindungsgemäßen Verfahrens (1) werden im Folgenden anhand der Figuren näher erläutert. Die Figuren zeigen schematische Darstellungen unterschiedlicher bevorzugter Ausführungsformen der in dem erfindungsgemäßen Verfahren verwendeten Mikrostruktur.
Figur 1 zeigt schematisch den generellen Aufbau einer mikrostrukturierten Kanalstruktur.
Figur 2 zeigt den funktionalen Aufbau einer mikrostrukturierten Kanalstruktur mit aktiven Bauelementen.
Figur 3 zeigt schematisch eine mikrostrukurierte Kanalstruktur mit einem kombinierten Assayfluidzugabe- und Detektionsbereich.
Figur 4 zeigt schematisch eine mikrostrukturierte Kanalstruktur, deren Reaktionsbereiche mit einzeln steuerbare Reaktionskanäle ausgestattet sind.
Figur 5 zeigt schematisch eine Mikrostruktur, eingesetzt für die Selektion einer sequenzspezifischen Endonukleaseaktivität.
Figur 6 zeigt in Seitenansicht den detaillierten Aufbau der in Figur 5 gezeigten Mikrostruktur, eingesetzt für die Selektion von sequenzspezifischer Endonukleaseaktivität.

### Detaillierte Beschreibung der Erfindung und der Figuren

"Mikrostruktur" im Sinne der vorliegenden Erfindung bezeichnet dreidimensionale Gebilde mit Kanalstruktur. Die Abmessungen der Kanalstrukturen liegen vorzugsweise im Bereich von 0,1 µm bis 100 µm Breite, besonders bevorzugt zwischen 1 und 10 µm. Die Aspektverhältnisse liegen vorzugsweise im Bereich von 0,1 bis 10, besonders bevorzugt bei 1.

"Fluide" im Sinne der vorliegenden Erfindung sind Flüssigkeiten oder Gase. Im einzelnen sind Testfluide zur Bildung der Genotypenkompartimente wässrige Lösungen oder Suspensionen komplexer Zusammensetzung, welche neben DNA alle weiteren essentiellen Komponenten zur zellfreien *in-vitro*-Expression des Genotyps in dem Phänotyp enthalten.

Die zur "zellfreien Expression geeigneten Expressionshilfsstoffe" im Sinne der Erfindung (auch "essentielle Komponenten" genannt) entstammen zellulären Transkriptions- und/oder Translationssystemen und umfassen Komponenten wie Translationsfaktoren (Initiations-, Elongations-, Terminationsfaktoren), Ribosomen (70S oder 80S), t-RNAs, Aminoacyl-tRNA-Synthasen. Zellfreie Systeme sind durch Zentrifugation und andere Aufreinigungstechniken gewonnene Zellextrakte, die über biologische Aktivität verfügen. Grundsätzlich können Zelllysate aus allen Zellen hergestellt werden. Für zellfreie in vitro-Expression können verschiedene prokaryotische und eukaryotische Zelllysate verwendet werden. Bevorzugt eingesetzt werden Extrakte aus *E.coli*-Zellen (z.B. S30-Extrakt), aus Reticulocyten ("Rabbit Reticulocytes") und aus Weizen-Keimlingen ("Wheat Germ"). Der Zellaufschluß wird nach dem Fachmann bekannten Protokollen durchgeführt (siehe dazu u.a. Promega Corporation, Protocols and Application Guide, Third Edition, 1996, und darin zitierte Literatur). Dem Fachmann ist ebenso bekannt, dass für eine effiziente Expression noch Zusätze weiterer Komponenten, wie z.B. Nukleotide, Aminosäuren, Energieäquivalente, Energie-regenerierende Systeme, Kofaktoren wie Mg²⁺, Pufferzusätze, evtl. exogene RNA-Polymerase wie T7-RNA-Polymerase notwendig sind.

Der zu untersuchende und zu exprimierende Genotyp kann in Form unterschiedlicher Matrizen zugesetzt werden, wie zirkuläre oder lineare DNA oder m RNA, jeweils zellulärer Herkunft oder in synthetischer Form.
Ausgangstemplate und Expressionssystem sollten aufeinander abgestimmt sein. So werden z.B. für die Umsetzung von DNA bevorzugt gekoppelte Transkriptions/Translationssysteme wie S30-Extrakt aus *E.coli* eingesetzt.

Für die Herstellung von DNA-Varianten (Genotyp-Bibliotheken) können verschiedene Methoden herangezogen werden.
Bekannte *in-vitro* Verfahren sind "Random Nucleic Acid Mutagenesis" erreicht z.B. durch Einsatz von Polymerasen mit hoher Fehlerquote (WO 9218645), Kassettenmutagenese (A. R. Oliphant et al., Gene 44 177-183 (1986); M. S. Horwitz, et al., Genome 31 112-117 (1989)), error-prone PCR (R. C. Cadwell und G. F. Joyce, PCR Methods Appl. 2 28-33 (1992)), und "Site Saturation Mutagenesis". Des Weiteren geeignet ist der Einsatz von "Recombination Chain Reaction" (WO 0134835), "DNA-Shuffling" (WO 9522625), "Staggered Extension Method" (WO 9842728), oder "Random Priming Recombination" (WO9842728). Eingesetzt werden können auch nicht-homologe Rekominationsmethoden wie "Itchy" (M. Ostermeier et al., Nature Biotechnology 17 1205-1209 (1999)).

Zur Bildung der Trennmedienkompartimente werden nicht wassermischbare Fluide eingesetzt. Dies sind insbesondere hydrophobe, inerte organischchemische Substanzen welche bei Betriebs-Raumtemperatur und Betriebsdruck als flüssige Phase vorliegen. In bevorzugter Form werden aliphatische oder aromatische Kohlenwasserstoffe, höhere Alkanole oder Alkanone, Ester oder Ether höherer Kohlenwasserstoffe, halogenierte Kohlenwasserstoffe, Mineralöle, Silikonöle oder Mischungen aus diesen Substanzen eingesetzt.

Als Assayfluid wird in bevorzugter Ausführungsform eine die Nachweisreagenzien enthaltende wässrige Lösung, Suspension oder Emulsion eingesetzt.
Bevorzugte Nachweisreagenzien sind Substrate, die durch Interaktion mit dem aus dem Genotyp exprimierten Phänotyp eine messbare Veränderungen erfahren. Messbare Veränderungen umfassen dabei u.a. Änderungen im Absorptionsspektrum, Änderungen der chromophoren, fluorophoren Eigenschaften, Veränderungen der messbaren Radioaktivität..

Eingesetzt werden können auch Mischungen an Substraten und Reagenzien, die startend mit dem durch den exprimierten Phänotyp veränderten ersten Substrat in einer Reaktionskaskade stöchiometrisch zu einem messbaren Endprodukt führen.

Bevorzugt werden Substrate eingesetzt, die mit definierten funktionellen, und damit definiert nachweisbaren Gruppen gekoppelt sind. Beispiele für derartige Gruppen sind fluorophore Marker wie z. B. Rhodamine Green (Molecular Probes Inc., Oregon, USA) oder Cy-5 (Amersham Biosciences Europe GmbH, Freiburg).

Die Substrate sind dabei stets entsprechend des exprimierten Phänotyps und dessen Aktivitätsspektrums zu wählen. Beispielsweise kann für die Selektion von exprimierten Proteasevarianten ein mit einem Fluorophor gekoppeltes Peptidsubstrat eingesetzt werden.

Die Gestaltung des Assayfluids bestimmt die Selektionsparameter für einen positiv zu beurteilenden Phänotyp, und bestimmt damit auch die Auslese der entsprechend dazugehörigen Genotypen.

Die Flussgeschwindigkeit in den Kanälen des mikrostrukturierten Reaktionssubstrats beträgt in bevorzugter Ausführungsform zwischen 10⁻⁷ m s¹⁻ und 10⁻² m s⁻¹, in besonders bevorzugter Ausführungsform zwischen 10⁻⁶ m s⁻¹ und 10⁻⁴ m s⁻¹.

Trennung der Genotypen in einzelne Kompartimente: In bevorzugter Ausführungsform enthält jedes Kompartiment einen Genotyp. Die Segregierung der Genotypen kann dabei durch rein statistisches Verteilen oder auch gezielt durch direkte messtechnische Erfassung und entsprechende Vereinzelung der den Genotyp tragenden Biomoleküle erfolgen. Die den Genotyp tragenden Biomoleküle bestehen in der Regel aus doppelsträngiger DNA, ggfs. auch aus einzelsträngiger DNA oder RNA. In einer weiteren Ausführungsform werden mehrere Genotypen in einem Kompartiment zusammengefasst, um eine höhere Variantentenzahl untersuchen zu können. Ausgehend vom Genotyp wird innerhalb des Kompartiments der entsprechende Phänotyp gebildet. Die Segregierung erfüllt dabei sowohl die Aufgabe der Trennung der Vielzahl von Phänotypen zur Messung der Eigenschaften als auch der Kopplung von Genotyp und Phänotyp, welche eine anschliessende Isolierung verbesserter Genotypen ermöglicht. Die Kompartimentvolumina liegen allgemein im Bereich zwischen 0,01 und 10.000 fl, vorzugsweise im Bereich zwischen 0,1 und 1000 fl, besonders bevorzugt zwischen 1 und 100 fl.

Zugabe von Assayreagenzien in die einzelnen Kompartimente: Geeignete Reagenzien zur Detektion des Phänotyps können in der Mehrzahl der Anwendungen nicht bei der Bildung der Genotyp-Kompartimente zugegeben werden, da diese zum einen mit in-vitro-Verfahren zur Expression des Phänotyps interferieren können und da andererseits eine exakte zeitliche Kontrolle des Reaktionsverlaufs durch die frühe Zugabe der Reagenzien erschwert wird.

Detektion des Phänotyps: Die Bestimmung der phänotypischen Eigenschaften jeden Genotyps bzw. jedes Kompartiments erfolgt vorzugsweise mit optischen, besonders bevorzugt mit fluorimetrischen Verfahren. Geeignete Messverfahren zur Messung in Strukturabmessungen bis zu wenigen 100 nm sind beispielsweise beschrieben in DE 197 57 740.

Die Auslese von Genotypen: Die Auslese der Genotypen mit positiv beurteiltem Phänotyp wird primär durch Selektion der dem positiven Phänotyp entsprechenden Kompartimente erreicht. Diese Differenzierung in positive bzw. negative Phänotypen enthaltende Kompartimente wird durch eine der Detektion nachfolgend räumliche Auftrennung in entsprechende Selektionsreservoire erreicht.

Der Genotyp wird in Form der DNA oder RNA aus den einzelnen selektierten Kompartimenten isoliert und kann dem Prozess erneut zugeführt werden Dies ermöglicht einen neuen Zyklus einer zielgerichteten Auslese (bei den z.B. Assayansätze mit geänderter Zusammensetzung verwendet werden).

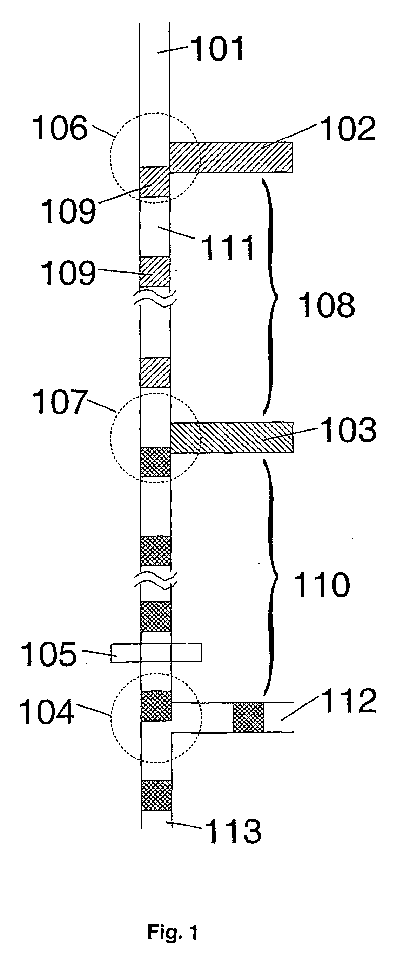

Schematisch ist der Aufbau einer mikrostrukturierten Kanalstruktur, welche diese Funktionen ineinander vereint und so die gerichtete Evolution von Biomolekülen ermöglicht, in Fig. 1 wiedergegeben.

Die Trennung der Kompartimente zur Segregierung der Genotypen erfolgt durch intermittierende Zugabe mindestens eines die Genotypen enthaltenden Testfluids (102) und mindestens eines Trennfluids (101) in einer Kompartimentierungsstruktur (106). Das Fluid (102) enthält dabei vorzugsweise alle zur Expression des Genotyps notwendigen Substanzen, die Zusammensetzung solcher Fluide und Verfahren zu deren Herstellung sind dem Fachmann bekannt (Lesley, S. A., Methods Mol. Biol. 37, 265 (1995)). Das Trennfluid kann sowohl eine wässrige Lösung als auch eine nicht-wässrige, vorzugsweise nicht wassermischbare Flüssigkeit oder ein Gas sein. Auch können zur Trennung der Genotypkompartimente (109) in sich strukturierte Trennfluidkompartimente (111) verwendet werden, die die Zugabe mehrerer Trennfluidkomponenten notwendig machen.
Die Temperaturen der beiden Reaktionsbereiche I (108) und II (110) können dabei durch geeignete Thermostatisierungselemente gesteuert werden. Beide Reaktionsbereiche (108 und 110) können anwendungsabhängig bei gleicher oder unterschiedlicher Temperatur betrieben werden.
Die Verwendung nicht wassermischbarer Fluide oder Gase als Trennfluid (101) in Kombination mit hydrodynamischen Fluss im mikrostrukturierten Reaktionssubstrat ist vorteilhaft, da durch diese Segregierung der Genotypkompartimente diffusiver Stofftransport zwischen Genotypkompartimenten (109) und zwischen Genotypkompartimenten (109) und Trennfluid (101) minimiert werden kann. Weiter ist vorteilhaft, dass durch die Verwendung nicht wassermischbarer Fluide oder Gase als Trennfluid (101) im hydrodynamischen Fluss die Axialdispersion minimiert wird. Die Expression des Genotyp erfolgt im Reaktionsbereich I (108). Die Reaktionszeit kann durch Experimentator durch Wahl der Länge des mikrostrukturierten Reaktionskanals im Reaktionsbereich I sowie durch Wahl der Fluidgeschwindigkeit im Reaktionsbereich I frei gewählt werden. Die Zugabe von Reaktionskomponenten zur Bestimmung phänotypischer Eigenschaften der im Genotypkompartiment (109) im Reaktionsbereich (108) gebildeten Biomoleküle wird in einem Bereich (107) des mikrostrukturierten Substrats eine durch den Experimentator gewählte Menge eines Assay-Fluids (103) mit den Genotypkompartimenten (109) vereinigt. Vorzugsweise besteht das Assay-Fluid (103) aus einem Fluid, das mit dem Genotypkompartimenten (109) mischbar oder in diesem löslich ist. Die Umsetzung der mit dem Assayfluid (103) zugesetzten Reaktionskomponenten durch die im Genotypkompartimenten (109) vorliegenden Biomoleküle erfolgt im Reaktionsbereich II (110). Die Reaktionszeit kann durch den Experimentator durch Wahl der Länge des mikrostrukturierten Reaktionskanals im Reaktionsbereich II sowie durch Wahl der Fluidgeschwindigkeit im Reaktionsbereich II frei gewählt werden.

Die Messung der aus der Umsetzung der Komponenten aus Assayfluid (103) und Genotypkompartiment (109) hervorgehenden Reaktionsprodukte erfolgt in einem Messbereich (105) des Reaktionssubstrats. Vorzugsweise werden zur Messung spektroskopische Messverfahren, höchst vorzugsweise konfokale fluoreszenzspektroskopische Methoden eingesetzt. Solche Methoden sind in der Lage in Strukturenabmessungen von wenigen 100 nm mit hoher Sensitivität die Probenzusammensetzung zu bestimmen. Die Anwendung konfokaler Detektionsverfahren zur Detektion kleinster Substanzmengen ist beispielweise in DE4301005 und WO9535492 gezeigt.

Genotypkompartimente mit im Sinne des Entwicklungsziels positiven Messergebnissen müssen von solchen mit negativen Messergebnissen getrennt werden, um vorteilhafte Varianten der eingesetzten Bibliothek von nachteiligen zu trennen. Diese Trennung erfolgt in einem Selektionsbereich (104) des Reaktionssubstrats durch gesteuerte Lenkung der Genotypkompartimente in einen von mindestens zwei Selektionskanälen 112 und 113.

Alle genannten Prozessschritte werden in durch Mikrostrukturtechnik gefertigten Kanalstrukturen vorteilhaft zusammengeführt und integriert. Derartige Kanalstrukturen können aus verschiedenen Werkstoffen gefertigt werden. Dazu zählen Metalle (z.B. Silizium), amorphe Werkstoffe (z.B. Glas), keramische Werkstoffe und polymere Werkstoffe (z.B. Polyurethane (PU), Polydimethylsiloxane (PDMS) und Polymethylmetacrylate (PMMA)). Bevorzugt werden die Kanalstrukturen durch Abscheidungs- oder Abtragungstechniken in metallischen, keramischen oder amorphen Materialien hergestellt. Vorteile dieser Ausführungsformen sind die geringen erreichbaren Toleranzen der Strukturen sowie eine hohe Funktionalität der integrierbaren Bauelemente. Daneben erlauben softlithografische Verfahren und Abformungsverfahren die Herstellung von mikrostrukturierten Reaktionssubstraten aus polymeren Materialien wie Polyurethanen (PU) und Polydimethylsiloxanen (PDMS). Da integrierte Funktionselemente innerhalb mikrostrukturierter Reaktionssubstrate gegeneinander fixiert sind ergeben sich hier gegenüber anderen Lösungsansätzen wesentliche Vorteile in Bezug auf die Stabilität des Systems.

Aktive Bauelemente können als Bestandteil der Mikrostruktur als Formgedächtniselemente, piezoelektrische Baugruppen oder magnetostriktive Elemente ausgeführt werden. In bevorzugter Ausführung werden aktive Bauelemente als Formgedächtniselemente ausgeführt. Zu den integrierbaren Bauelementen zählen beispielsweise Ventile und Pumpen (T. Gerlach, M. Schuenemann und H. Wurmus, Journal of Micromechanics & Microengineering. 5(2):199-201 (1995 Jun.)).

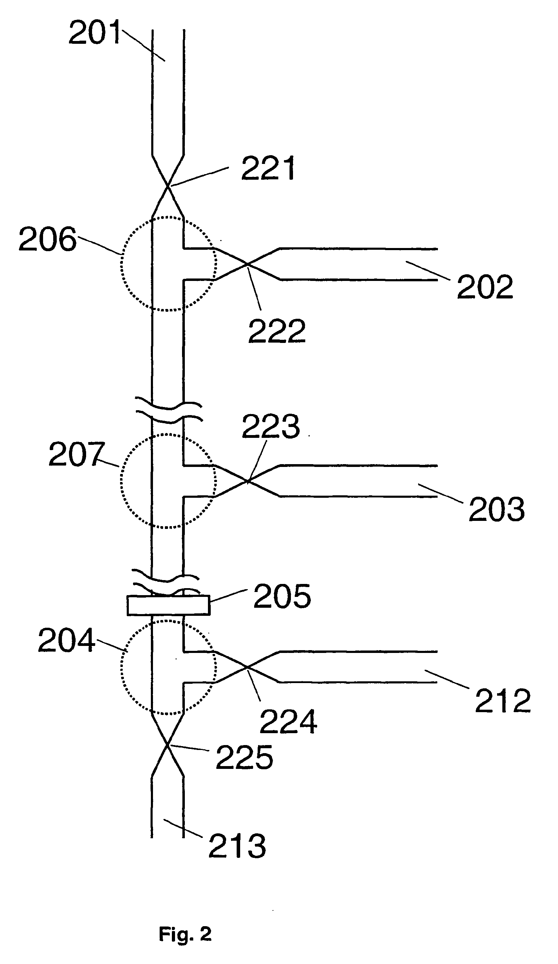

Aktive Bauelemente werden in bevorzugter Ausführungsform zur Segregierung im Fluidstrom und zur Selektion ausgewählter Kompartimente eingesetzt. In einer besonderen Ausführungsform sind die aktiven Bauelemente außerhalb der Mikrostruktur, aber mit dieser direkt verbunden, angeordnet.
In einer weiteren besonders bevorzugten Ausführungsform befinden sich die aktiven Bauelemente in der Mikrostruktur, d. h. sind Bestanteile.
Der funktionale Aufbau einer im erfindungsgemäßen Verfahren verwendeten mikrostrukturierten Kanalstruktur mit aktiven Bauelementen 221, 222, 223, 224 und 225 ist in Fig. 2 wiedergegeben. Die mikrostrukturierten Ventilelemente 221 und 222, die mit dem ersten bzw. zweiten Zuführkanal verbunden sind, dienen zur Bildung des beschriebenen kompartimentierten Fluidstroms aus den Fluidkomponenten 201 und 202 im Kompartimentierungselement 206.

Die Ventilelemente werden in bevorzugter Ausführungsform in einer durch den Experimentator festgelegten Abfolge und für eine durch den Experimentator festgelegte Zeitspanne geöffnet und gestatten damit den Durchtritt von Fluidelementen definierten Volumens.

Das Ventilelement 223 (das entsprechend den Ventilelementen 221 und 222 ausgebildet sein kann) steuert die Zugabe des Assayfluid 203 im Bereich 207. In bevorzugter Ausführungsform wird die Steuerung des Ventilelements mit der Steuerung der Ventilelemente 221 und 222 so koordiniert, dass Assayreagenzien dann zugegeben werden, wenn sich ein Genotypenkompartiment innerhalb des Zugabebereiches 207 befindet. Insbesondere bei Verwendung kompressibler Fluide oder mikrostrukturierter Reaktionssubstrate aus elastischen Materialien kann die Koordinierung der Ventilelemente 221, 222 und 223 unzureichend sein, um die Zugabe der Assayreagenzien zu einem Genotypenkompartiment im Zugabebereich 207 sicher zu gewährleisten. In weiterer bevorzugter Ausführungsform kann dann der Transport eines Genotypenkompartiments in den Zugabebereich 207 messtechnisch bestimmt und das Ventilelement 223 ausgehend von diesem Messwert geöffnet werden. Bevorzugt werden zur Detektion des Genotypenkompartiments im Zugabebereich 207 optische Messverfahren eingesetzt.
Die Ventilelemente 224 und 225 (Fig. 2) steuern die Selektion der Genotypenkompartimente nach Bestimmung deren phänotypischer Eigenschaften im Detektionsbereich 205. Zur Selektion einzelner Genotypenkompartimente werden die Ventilelemente der mindestens zwei Selektionskanäle 212 und 213 alternativ geöffnet.

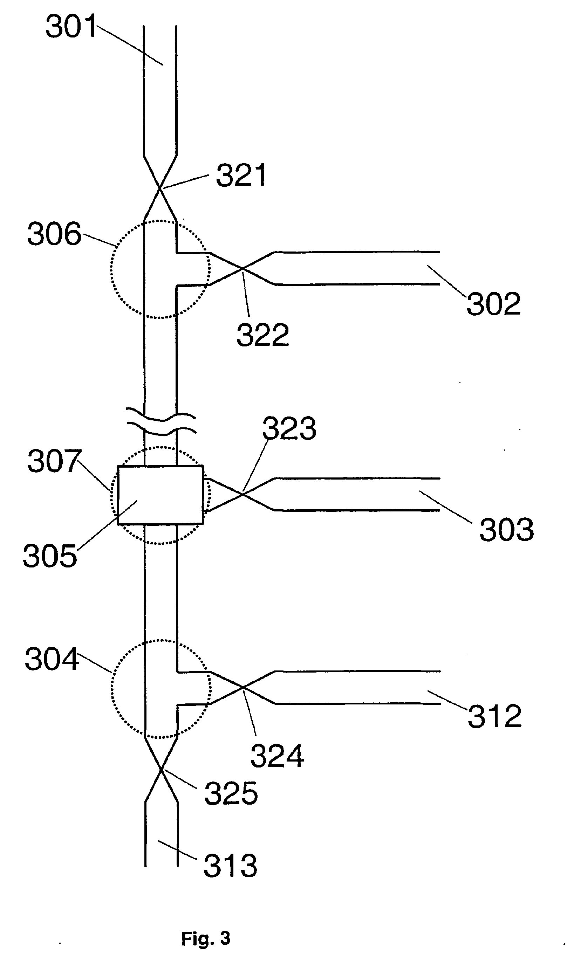

In einer weiteren bevorzugten Ausführungsform, wie in Fig. 3 schematisch dargestellt ist, können phänotypische Eigenschaften des gebildeten Genprodukts in direkter Umgebung des Assayfluidzugabebereichs (307) bestimmt werden. Das mikrostrukturierte Reaktionssubstrat wird unter Auslassung des Reaktionsbereichs II (vergleiche dazu Fig. 1, Reaktionsbereich II (110)) dann so hergestellt, dass Assayfluidzugabebereich (307) und Detektionsbereich (305) örtlich zusammenfallen. Vorteile dieser Ausführungsform sind, dass schnelle Umsetzungen zwischen Genprodukt und Assayreagenzien so als zeitlich aufgelöste Messreihen beobachtet werden können. Solche Zeitreihenmessungen gestatten für schnelle Umsetzungen eine bessere Charakterisierung phänotypischer Eigenschaften des gebildeten Genprodukts als die Bestimmung eines einzelnen Messwerts nach einer durch die Wahl der Länge des Reaktionsbereichs II gegebenen festgelegten Reaktionszeit. Die Dauer der Zeitreihenmessung kann anwendungsabhängig durch Unterbrechung des Fluidstroms im mikrostrukturierten Reaktionssubstrat durch gleichzeitiges Schließen der Ventilelemente 321, 322 und 323 verlängert werden.

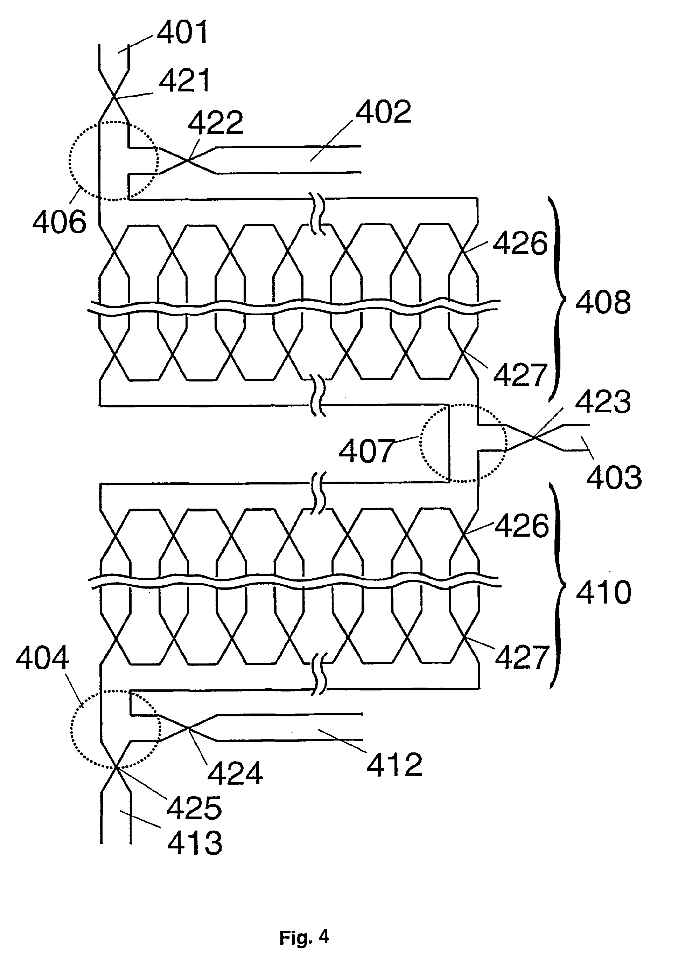

Die Aufenthaltszeit von Probenkompartimenten in den Reaktionsbereichen 108 und 110 des in Fig. 1 schematisch dargestellten Reaktionssubstrats ist gegeben durch die Länge des Reaktionsbereichs und die Fluidgeschwindigkeit in diesem Bereich. Nach dem Fachmann bekannten Zusammenhängen steigt bei hydrodynamischen Fluidtransport der Druckverlust mit der Länge der Kanalstruktur. Für sehr lange Reaktionszeiten und den damit notwendigen langen Reaktionsbereichen kann der zum hydrodynamischen Transport erforderliche Druck ein technisches Hemmnis zur Konstruktion des mikrostrukturierten Reaktionssubstrats sein. In diesem Fall stellt die in Fig. 4 skizzierte Ausführungsform eine vorteilhafte Lösung des technischen Problems dar. Die Reaktionsbereiche 408 und 410 sind hier ausgeführt als getrennte, durch Öffnen einzelner Ventilelemente 426 ansteuerbare Reaktionskanäle 427. Im erfindungsgemäßen Verfahren werden so einzelne Kanäle nach Öffnen einzelner Ventile mit dem kompartimentierten Fluidstrom befüllt. Die Vielzahl vorgesehener Reaktionskanäle 426 kann so befüllt werden. Nach Ablauf einer frei wählbaren Reaktionszeit kann das in den einzelnen Reaktionskanälen vorliegende kompartimentierte Fluid durch weiteres kompartimentiertes Fluid oder durch ein nicht kompartimentiertes Fluid verdrängt und dem jeweils nächsten Funktionselement (407) oder (404) zugeleitet werden.

Erfindungsgemäß ist das Verfahren geeignet, Biomoleküle mit bestimmten phänotypischen Eigenschaften aus einer Vielzahl von Varianten zu selektieren. Die Varianten können durch *in-vitro*-Verfahren zur Mutation einer DNA-Sequenz des Ausgangsphänotyps hergestellt werden Besonders ist das Verfahren für die Selektion von Phänotypen mit nicht zellkompatiblen Genotypen geeignet. Nicht zellkompatibel ist beispielsweise eine sequenzspezifische Endonukleaseaktivität, wenn der Zelle die Methylaseaktivität mit entsprechender Sequenzspezifität fehlt, da hier zelleigene DNA durch die katalytische Aktivität des exprimierten Proteins geschädigt wird. Ein solcher nicht zellkompatibler Phänotyp findet sich weiterhin beispielsweise dann, wenn die katalytische Aktivität eines exprimierten Proteins toxische Wirkungen auf den zellulären Stoffwechsel oder andere wachstumshemmende Eigenschaften hat.

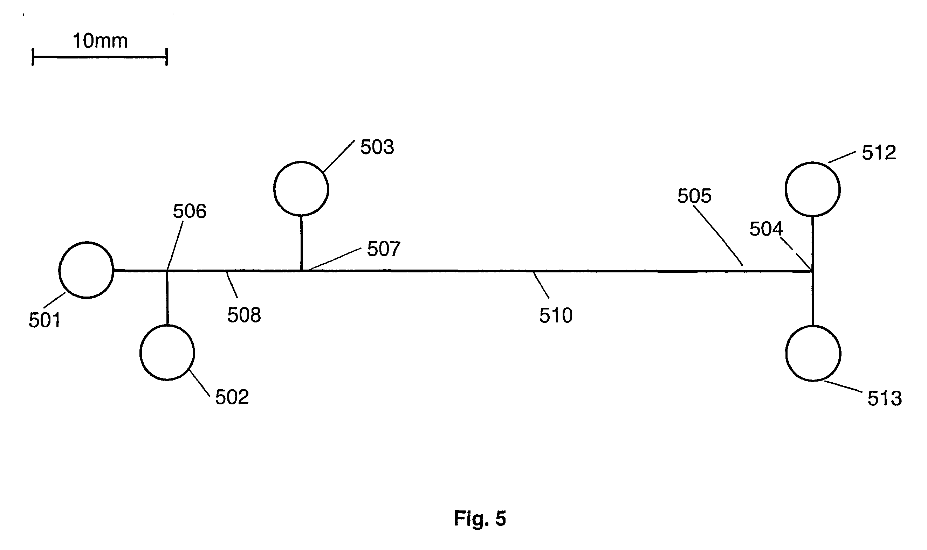

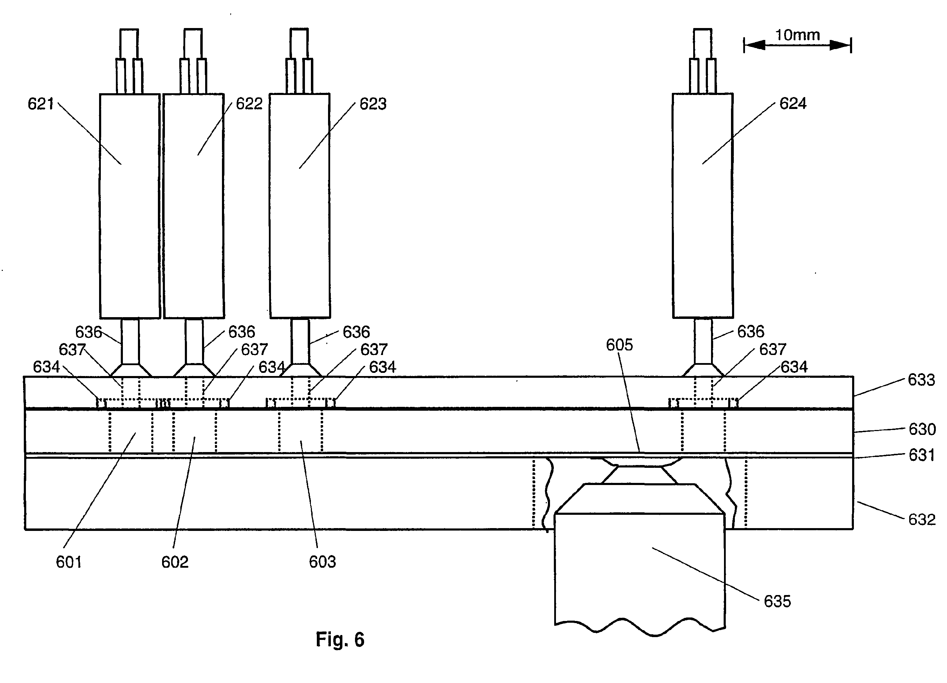

Für die Selektion einer spequenzspezifischen Endonukleaseaktivität kann, wie nachfolgend im Beispiel beschrieben ist, ein Mikrostruktur-Design entsprechend Fig. 5 und Fig. 6 eingesetzt werden. In Fig. 5 ist eine Mikrostruktur gezeigt, die aus den Reagenzienzuführungen 501 (Trennfluid), 502 (Expressionsfluid), 503 (Assayfluid), der Kompartimentierungsstruktur 506, dem Assayzugabebereich 507, dem Selektionsbereich 504, dem Meßbereich 505 sowie den Reservoiren 512 bzw. 513 für selektierte bzw. verworfene Kompartimente besteht. Die Bereiche zwischen Kompartimentierungsstruktur 505 und Assayzugabereich 507 bzw. zwischen Assayzugabebereich 507 und Messbereich 505 stellen jeweils Reaktionsbereiche I bzw. II (508 bzw. 510) dar. In diesen Bereichen kann die Expression in den Phänotyp bzw. die Reaktion des Phänotyps mit den zugegebenen Assayreagenzien stattfinden.

Der Aufbau des vollständigen mikrostrukturierten Selektionsmoduls in der Seitenansicht ist in Figur 6 wiedergegeben. Die Mikrostruktur 630 ist durch ein Deckglas 631 (Floatglas; Stärke 170 um) verschlossen. Die Ventilelemente 621, 622, 623, 624, 625 (in Abb. 6 verdeckt) sind als Miniaturventile (Lee Hydraulische Miniaturkomponenten GmbH, Frankfurt am Main,) ausgeführt. Diese Mikroventile werden durch eine externe Steuereinheit (Eigenbau, Schaltungsaufbau gemäß Herstellerangaben Lee GmbH) geschaltet. Die Ventilelemente sind in einer Trägerstruktur 633 fixiert, die durch Anpressen an die Mikrostruktur 630 mit dieser verbunden wird. Die hydraulisch dichte Verbindung von Mikroventilen (621, 622, 623, 624, 625) über die Verbindungselemente 636 und die Durchführungen 637 mit den Reservoiren 601, 602, 603, 612, 613 der Mikrostruktur 630 wird durch die Dichtungselemente 634 erreicht. Zur Herstellung der mechanischen Festigkeit des vollständigen Selektionsmoduls wird die Mikrostruktur 630 auf einem Träger 632 gelagert. Der Träger 632 dient ferner zur Temperierung des Selektionsmoduls. Zur optischen Detektion des biochemischen Umsatzes der Reaktion in den Fluidkompartimenten (wie in Fig.1 unter Nummer 109 schematisch dargestellt) wird im Detektionsbereich 605 ein Mikroskopobjektiv (635) an die mit einem Deckglas verschlossene Mikrostruktur herangeführt.

Das erfindungsgemäße Verfahren und die darin verwendete Mikrostruktur wird anhand des nachfolgenden, nicht einschränkenden Beispiels näher erläutert.

### Beispiel

Das hier angeführte Beispiel beschreibt die Selektion einer sequenzspezifischen Endonukleaseaktivität.

Aufbau und Zusammensetzung einer Mikrostruktur: Für die Selektion einer sequenzspezifischen Endonukleaseaktivität wird eine Mikrostruktur entsprechend Fig. 5 und. Fig. 6 gewählt. Die Kanalstrukturen solch einer Mikrostruktur werden durch Vakuum-UV-Ablation aus dem Material PMMA (Polymethylmethacrylat) hergestellt. Die Herstellung und Verwendung solcher Mikrostrukturen zur Analyse biochemischer Reaktionen ist wiederholt gezeigt worden (M. Lapczyna, Dissertation Universität Gesamthochschule Kassel "Vakuum-ultraviolett (VUV)-Laser-induzierte Mikrostrukturierung von Polymer-Substraten für laserspektroskopische Anwendung in der Bioanalytik" (1998); K. Dörre, Dissertation Technische Universität Braunschweig "Machbarkeitsstudien zur DNA-Einzelmolekülsequenzierung in Mikrostrukturen" (2000), ; K. Dörre et al., Journal of Biotechnology 86 225-236 (2001); J. Stephan et al., Journal of Biotechnology 86 255-267 (2001)). Die Kanalbreite und -tiefe beträgt etwa 1 µm. Die Reservoire 601, 602, 603, 612 und 613 werden durch feinmechanische Bearbeitung der Struktur eingebracht.

Mit den in DE 19757740 beschriebenenfluoreszenzspektroskopischen Verfahren lassen sich auch in mikrostrukturierten Reaktionskanälen biochemische Umsetzungen mit hoher Auflösung bestimmen. Weiter ermöglicht die Mikroskopoptik die visuelle Kontrolle der Kompartimentierung innerhalb der Mikrostruktur und damit die experimentelle Anpassungen der notwendigen Betriebsparameter wie Vordruck der Reagenzienzuführungen sowie Dauer und Koordinierung der Schaltintervalle der Ventilelemente 621, 622, 623, 624, 625.

Selektion auf sequenzspezifische Endonukleaseaktivität: Vor der Verwendung des Expressionsmoduls wird die Kanalstruktur mit Trennfluid gefüllt. Vor der Verwendung des Expressionsmoduls wird die Kanalstruktur mit Trennfluid gefüllt.

Als Trennfluid wird ein Gemisch perfluorierter aliphatischeer Kohlenwasserstoffe (Fluorinert FC40, Art. Nr. F9755, Sigma Aldrich GmbH, Deisenhofen) eingesetzt. Das Trennfluid verhält sich gegenüber biochemischen Reaktionen inert. Das Trennfluid wird dazu in das Kompartiment 501 eingefüllt. Das Trennfluid wird dazu in das Kompartiment 501 eingefüllt. Die Befüllung der mikrostrukturierten Kanäle erfolgt z.T. spontan durch Kapillarwirkung. Nicht spontan gefüllte Teilbereiche der Kanäle können durch Anlegen von Unterdruck an die Kompartimente 502, 503, 512, 513 befüllt werden. Nachfolgend werden alle notwendigen Reagenzien in die vorgesehenen Kompartimente der Mikrostruktur eingebracht (Expressionsfluid 502, Assayfluid 503). Differenzvolumina zur Füllung des gesmaten Kompartimentvolumens werden mit einem nicht wassermischbaren Kopplungsfluid (niederviskoses Mineralöl, Art. Nr. M5904, Sigma Aldrich GmbH, Deisenhofen) aufgefüllt. Ebenfalls werden die Reservoire 512 und 513 mit Kopplungsfluid befüllt. Das Kopplungsfluid dient der hydraulischen Kopplung zwischen Ventilelementen und den Fluidreservoiren durch ein nichtkompressibles Medium. Das wässrige Expressionsfluid und das Assayfluid werden mit dem Kopplungsfluid überschichtet. Die Ventilelemente 622 bis 625 werden mit kopplungsgefüllten, druckbeaufschlagten Reservoiren verbunden, das Ventilelement 621 wird mit trennfluidgefülltem, druckbeaufschlagtem Reservoir verbunden. Der Vordruck jedes Reservoirs wird dabei getrennt für jedes Reservoir gewählt.

Durch Öffnen der Ventile 621, 622, 623, 624, 625 werden die Schlauchverbindungen sowie die Ventile selbst mit Trennfluid bzw. Kopplungsfluid ohne Einschluss von Gasblasen gefüllt. Das Trägermodul 633 wird wiederum ohne Einschluß von Gasblasen durch Anpressen mit der Mikrostruktur 630 verbunden.
Das Expressionsfluid (502) enthält einen zur zellfreien Proteinexpression geeigneten E.-coli-S30-Extrakt. Das Expressionsfluid (502) enthält einen zur zellfreien Proteinexpression geeigneten E.-coli-530-Extrakt inklusive aller weiterer Hilfsstoffe (T7-RNA-Polymerase etc.) (Lesley, S.A., Methods Mol. Biol. 37, 265 (1995)).

Die Genotypenbibliothek , basierend auf dem Gen EcoRI aus E. coli, wird in diesem Ansatz bei einer Temperatur von 4 °C auf eine Konzentration von 500 pM Plasmid-DNA verdünnt.

Die Ventilelemente (621 und 622) des Kompartimentierungselements (506) des mikrostrukturierten Reaktionssubstrats werden bei Zugabe von Expressionsfluid (502 bzw. 602) und Trennfluid (501 bzw. 601) so gesteuert, dass sich wässrige Genotypkompartimente mit einer Länge von ca. 2 µm und Trennfluidkompartimente mit einer Länge von 10 µm bilden. In Kanalabmessungen von 1 µm x 1 µm beträgt das Volumen eines einzelnen Genotypenkompartiments entsprechend 2 fl, jedes Genotypenkompartiment trägt dann im statistischen Mittel ca. 0,6 DNA-Moleküle der eingesetzten Bibliothek. Entsprechend enthalten ca. 54 % der gebildeten Kompartimente kein DNA-Molekül, 33 % der Kompartimente ein DNA-Molekül und 13 % der Kompartimente zwei oder mehr DNA-Moleküle.
Es wurde gefunden, dass es durch unspezifische nukleolytische Aktivität häufig zu einem Verlust an eingesetzter DNA kommt, sodass die Konzentration der eingesetzten DNA anwendungsabhängig geringfügig höher im nanomolaren (1 - 10 nM) Bereich gewählt werden kann.
Die Transportgeschwindigkeit innerhalb des Inkubationsbereichs I (508) der Kanalstruktur wird mit ca. 2,0 cm h⁻¹ gewählt, sodass jedes gebildete Genotoypenkompartiment nach ca. 0,5 h die Inkubationsstrecke I von 1 cm zur Proteinexpression durchlaufen hat. Nach Expression des Phänotyps werden den Genotypenkompartimenten jeweils ca. 8 fl Assayfluids (503 bzw. 603) zudosiert. Das Assayfluid (503/603) enthält alle für die endonukleolytische Reaktion und ihren Nachweis nötigen Komponenten: 150 mM KOAc, 37,5 mM Tris-Acetat, ph 7,6, 15 mM MgOAc, 0,75 mM β-Mercaptoethanol, 515 µg/ml BSA, 0,05 % Triton X-100, 0,5 % Glycerin, 10 nM doppelt fluoreszenzgelabeltes DNA-Substrat. Entsprechend den in DE 19757740 genannten Methoden wird die Endonukleaseaktivität spezifisch durch Zusatz doppelt fluoreszenzmarkierter DNA Substrate bestimmt.
Als DNA-Substrat zum Nachweis der endonukleolytischen Reaktion werden entsprechend den von Winkler T. et al. beschriebenen Verfahren (Proc.Natl.Acad.Sci.USA 69 (1999) 1375-1376) fluoreszenzfarbstoffmarkierte Oligonucleotide verwendet. Die verwendeten Substrate sind nachfolgend dargestellt:

Cy5 (Amersham Biosciences Europe GmbH, Freiburg ) und RhG (Rhodamine Green von Molecular Probes Inc., Oregon, USA) sind typischerweise verwendete Fluoreszenzfarbstoffe. Die Nukleobasen sind nach dem Fachmann bekannter Nomenklatur mit den Buchstaben A,C,G,T abgekürzt. Die beiden Oligonucleotide I und II können nach Hitzedenaturierung zu einen Doppelstrang "annealt" werden, der beide Fluoreszenzfarbstoffe sowie die spezifische Schnittsequenz der Endonuclease EcoRI trägt. Die Schnittstelle der sequenzspezifischen Endonuklease EcoRI ist unterstrichen dargestellt.

Die Zugabe des Assayfluids (503 bzw. 603) erhöht sich die mittlere Fluidgeschwindigkeit auf 3,3 cm h⁻¹. Nach einer Inkubationsszeit von 1 h durchläuft jedes Genotypenkompartiment den Inkubationsbereich II (510) von 3,3 cm Länge zum Detektionselement (505 bzw. 605). Die Endonukleaseaktivität wird gemäß den in T. Winkler et. al beschriebenen fluoreszenzspektroskopischen Verfahren (Proc.Natl.Acad.Sci.USA 69 1375-1378 (1999)) nachgewiesen. Solche Verfahren lassen sich auch in Mikrostrukturen anwenden wie in der oben zitierten Dissertation von K. Dörre gezeigt werden konnte. Am Selektionselement (504) erfolgt dann durch Steuerung der Ventilelemente (624 und 625) die Selektion positiv bewerteter Genotypenkompartimente.

Positiv bewertete Kompartimente werden so durch Öffnung der Ventilelements 625 in das Reservoir 512 abgelenkt, während negativ bewertete Kompartimente durch Öffnung des Ventilelements 624 in das Reservoir 513 abgelenkt werden.

Nach Beendigung des Selektionsvorgangs können im Verbindungskanal zwischen der Selektionsstruktur 504 und dem Reservoir 512 verbleibende Genotypenkompartimente durch dauerhaftes Schließen der Ventilelemente 622, 623 und 624 sowie dauerhaftes Öffnen der Ventilelemente 621 und 625 in das Reservoir 512 gefördert werden.

Die Mischung aller positiv bewerteten Genotypenkompartimente wird nach Lösen der Verbindung zwischen Mikrostruktur 630 und Trägerstruktur 633 aus dem Kompartiment 512 entnommen. Da das Gesamtvolumen der im Reservoir 512 befindlichen Genotypenkompartiment gering ist , wird zur Entnahme der Genotypen ein zusätzliches Volumen von 10 ul Puffer (Tris-EDTA-Puffer, 50mM Tris(hydroxymethyl)-aminomethan (Merck KG, Art.Nr. 1.08382.2500), 10mM Titriplex III (Merck KG, Art.Nr. 1.08418.1000), pH 7.0 ) manuell auf den Boden des Reservoirs 512 pipettiert. Durch mehrfaches Aufnehmen und Abgeben des Volumens können die Genotypenkompartimente im Puffervolumen aufgenommen werden. Das Puffervolumen wird dem Kompartiment entnommen, gegebenenfalls überführte Reste von Trennfluid und Kopplungsfluid können durch Zentrifugation und durch Entnehmen von der wässrigen Phase abgetrennt werden.

Nach dem Fachmann bekannten Methoden (PCR Polymerasekettenreaktion) werden die im Puffervolumen befindlichen Genotypen vervielfältigt, so daß sie nachfolgenden molekularbiologischen Manipulationen und ggf. der wiederholten Zuführung in einen Expressions und Selektionszyklus zugänglich sind.

## Patentansprüche

1. Verfahren zur zellfreien Selektion von Genotyp-Varianten aus Genotyp-Bibliotheken in einer Mikrostruktur, umfassend die folgende Abfolge von Reaktionsschritten :
(a) Zusammenführen eines Testfluids, umfassend eine Genotypen-Bibliothek in einer exprimierbaren Form und für die zellfreie Expression geeignete Expressionshilfsstoffe, und eines Trennfluids in der Mikrostruktur, sodass einzelne Kompartimente des Testfluids entstehen;
(b) Transportieren der Kompartimente durch die Mikrostruktur, wobei die Expression des Genotyps in den Phänotyp in den Kompartimenten erfolgt,
(c) Detektieren des Phänotyps in den Kompartimenten und
(d) Selektieren der Kompartimente entsprechend ihrer Phänotypen.

2. Verfahren nach Anspruch 1, wobei nach Schritt (b) ein Assayfluid mit Reagenzien, die zum Nachweis des Phänotyps geeignet sind, zu den Kompartimenten zugegeben wird.

3. Verfahren nach Anspruch 1 oder 2, wobei
- das Testfluid und für die zellfreie Expression geeignete Expressionshilfsstoffe, ausgewählt sind aus wässrigen Lösungen und Suspensionen komplexer Zusammensetzungen, vorzugsweise einem zur in-vitro-Proteinexpression geeigneten Zellextrakt enthält, und/oder
- das Trennfluid ein nicht wassermischbares Fluid ist und insbesondere ausgewählt ist aus aliphatischen und aromatischen Kohlenwasserstoffen, höheren Alkoholen, höheren Alkanonen, Estern und Ethern höherer Kohlenwasserstoffe, halogenieren Kohlenwasserstoffen und Silikonen und Mischungen aus diesen Substanzen und/oder
- die Transportgeschwindigkeit der Kompartimente in der Mikrostruktur 1 x 10⁻⁷ bis 1 x 10⁻² m/s, vorzugsweise 1 x 10⁻⁶ bis 1 x 10⁻⁴ m/s beträgt.

4. Verfahren nach einem oder mehreren der Ansprüche 1 bis 3, wobei die Konzentration der Genotypen-Bibliothek und das Zusammenführen von Testfluid und Trennfluid so gewählt sind, dass im statistischen Mittel nur eine Genotypvariante pro Kompartiment enthalten ist, und/oder wobei das Kompartimentvolumen 0,01 fl bis 10 pl, vorzugsweise 0,1 fl bis 1 pl, besonders bevorzugt 1 bis 100 fl beträgt.

5. Verfahren nach einem oder mehreren der Ansprüche 2 bis 4, wobei das Assayfluid mit dem Testfluid mischbar und mit dem Trennfluid unmischbar ist, insbesondere ausgewählt ist aus wässrigen Lösungen, Suspensionen und Emulsionen, und der Zeitpunkt der Zugabe so gewählt ist, dass sich bereits eine für die Detektion hinreichende Menge Genprodukt gebildet hat.

6. Verfahren nach einem oder mehreren der Ansprüche 1 bis 5, wobei
- die Assayreagenzien für die zu selektierende Funktion spezifisch sind und insbesondere solche sind, die geeignet sind, die zu selektierende Funktion mit optischen, vorzugsweise fluorimetrischen Messverfahren zu analysieren und/oder
- das Detektieren des Phänotyps in den Kompartimenten die qualitative und/oder quantitative Bestimmung der phänotypischen Eigenschaften umfasst, und insbesondere durch optische, besonders bevorzugt durch fluorimetrische Verfahren erfolgt.

7. Verfahren nach einem oder mehreren der Ansprüche 1 bis 6, wobei der Phänotyp endonukleolytische Aktivität ist.

8. Verfahren nach einem oder mehreren der Ansprüche 1 bis 7, wobei das Selektieren der Kompartimente durch Sortieren erfolgt und/oder das Verfahren weiterhin den Reaktionsschritt
(e) Isolieren des Genotyps der aussortierten Kompartimente unter Bildung einer neuen Genotyp-Bibliothek umfasst.

9. Verfahren nach Anspruch 8, wobei die erhaltene Genotyp-Bibliothek einem oder mehreren weiteren Reaktionszyklen (a) bis (d) unterworfen wird.

## Claims

1. A method for the cell-free selection of genotype variants from genotype libraries in a microstructure, comprising the following sequence of reaction steps:
(a) combining a test fluid comprising a genotype library in an expressible form and expression aids suitable for cell-free expression with a separation fluid in the microstructure to form individual compartments of the test fluid;
(b) transporting the compartments through the microstructure, the expression of the genotype into the phenotype being effected in the compartments;
(c) detecting the phenotype in the compartments; and
(d) selecting the compartments in accordance with their phenotypes.

2. The method according to claim 1, wherein an assay fluid with reagents suitable for detecting the phenotype is added to the compartments after step (b).

3. The method according to claim 1 or 2, wherein
- said test fluid and expression aids suitable for cell-free expression are selected from aqueous solutions and suspensions of complex compositions, preferably a cell extract suitable for *in-vitro* protein expression; and/or
- said separation fluid is a water-immiscible fluid, especially selected from aliphatic and aromatic hydrocarbons, higher alcohols, higher alkanones, esters and ethers of higher hydrocarbons, halogenated hydrocarbons and silicones and mixtures of these substances; and/or
- the transport speed of the compartments within the microstructure is from 1 x 10⁻⁷ to 1 x 10⁻² m/s, preferably from 1 x 10⁻⁶ to 1 x 10⁻⁴ m/s.

4. The method according to one or more of claims 1 to 3, wherein the concentration of said genotype library and said combining of the test fluid and the separation fluid are selected in such a way that a statistic average of only one genotype variant is contained per compartment, and/or wherein the compartment volume is from 0.01 fl to 10 pl, preferably from 0.1 fl to 1 pl, more preferably from 1 to 100 fl.

5. The method according to one or more of claims 2 to 4, wherein said assay fluid is miscible with said test fluid and immiscible with said separation fluid, especially being selected from aqueous solutions, suspensions and emulsions, and the time of the addition is selected in such a way that an amount of gene product sufficient for detection will already have formed by then.

6. The method according to one or more of claims 1 to 5, wherein:
- the assay reagents are specific for the function to be selected, especially being those which are suitable for analyzing the function to be selected with optical, preferably fluorimetric, measuring methods; and/or
- the detection of the phenotype in the compartments comprises the qualitative and/or quantitative determination of the phenotypical properties and is effected, in particular, by optical, more preferably fluorimetric, methods.

7. The method according to one or more of claims 1 to 6, wherein said phenotype is manifested by endonucleolytic activity.

8. The method according to one or more of claims 1 to 7, wherein said selecting of the compartments is effected by sorting, and/or the method further comprises the reaction step of
(e) isolating the genotype of the selected compartments to form a new genotype library.

9. The method according to claim 8, wherein the genotype library obtained is subjected to one or more further reaction cycles (a) to (d).

## Revendications

1. Procédé de sélection acellulaire de variants de génotypes à partir de banques de génotypes dans une microstructure, comprenant le déroulement suivant d'étapes réactionnelles :
(a) combinaison d'un fluide de test, contenant une banque de génotypes sous une forme exprimable et des auxiliaires d'expression convenant à l'expression acellulaire, et d'un fluide de séparation dans la microstructure, de façon à obtenir des compartiments individuels de fluide de test ;
(b) transfert des compartiments à travers la microstructure, ce qui réalise l'expression du génotype en phénotype dans les compartiments ;
(c) détection du phénotype dans les compartiments, et
(d) sélection des compartiments en fonction de leurs phénotypes.

2. Procédé selon la revendication 1, dans lequel, après l'étape (b), un fluide d'analyse contenant des réactifs adaptés à la mise en évidence du phénotype, est ajouté aux compartiments.

3. Procédé selon la revendication 1 ou 2, dans lequel
- le fluide de test et les auxiliaires d'expression convenant à l'expression acellulaire sont choisis parmi des solutions et des suspensions aqueuses de compositions complexes, de préférence un extrait cellulaire adapté à l'expression de protéines *in vitro,* et/ou
- le fluide de séparation est un fluide non miscible à l'eau et est en particulier choisi parmi des hydrocarbures aliphatiques et aromatiques, des alcools supérieurs, des alcanones supérieurs, des esters et des éthers d'hydrocarbures supérieurs, des hydrocarbures halogénés et des silicones, ainsi que des mélanges de ces substances et/ou
- la vitesse de transfert des compartiments dans la microstructure est de 1 x 10⁻⁷ à 1 x 10⁻² m/s, de préférence de 1 x 10⁻⁶ à 1 x 10⁻⁴ m/s.

4. Procédé selon une ou plusieurs des revendications 1 à 3, dans lequel la concentration de la banque de génotypes et la combinaison du fluide de test et du fluide de séparation sont choisies de telle sorte que de manière statistique, seul un variant de génotype est contenu par compartiment et/ou dans lequel le volume de compartiment est de 0,01 fl à 10 pl, de préférence de 0,1 fl à 1 pl, de façon particulièrement préférée de 1 à 100 fl.

5. Procédé selon une ou plusieurs des revendications 2 à 4, dans lequel le fluide d'analyse est miscible avec le fluide de test, et non miscible avec le fluide de séparation, et est en particulier choisi parmi des solutions, des suspensions et des émulsions aqueuses, et dans lequel le moment de l'adjonction est choisi de telle sorte qu'une quantité de produit génétique suffisant à la détection s'est déjà formée.

6. Procédé selon une ou plusieurs des revendications 1 à 5, dans lequel :
- les réactifs d'analyse sont spécifiques de la fonction à sélectionner et sont en particulier des réactifs qui conviennent à analyser la fonction à sélectionner avec des procédés de mesure optiques, de préférence fluorimétriques, et/ou
- la détection du phénotype dans les compartiments comprend l'analyse qualitative et/ou quantitative des propriétés phénotypiques et s'effectue en particulier par des procédés optiques, de façon particulièrement préférée des procédés fluorimétriques.

7. Procédé selon une ou plusieurs des revendications 1 à 6, dans lequel le phénotype est une activité endonucléolytique.

8. Procédé selon une ou plusieurs des revendications 1 à 7, dans lequel la sélection des compartiments s'effectue par criblage et/ou le procédé comprend en outre l'étape réactionnelle (e) consistant à isoler le génotype des compartiments triés en formant une nouvelle banque de génotypes.

9. Procédé selon la revendication 8, dans lequel la banque de génotypes obtenue est soumise à un ou plusieurs autre(s) cycles(s) réactionnel(s) (a) à (d).
